# EUROPEAN PATENT SPECIFICATION

(11) **EP 1 079 183 B1**
(45) Date of publication and mention of the grant of the patent: **28.06.2006**
(21) Application number: 00202815.7
(22) Date of filing: 09.08.2000
(51) Int. Cl.: F24F 3/16, F24F 1/00

(54) **Self-sanitizing air-conditioning unit, and the relative sanitizing process**
Klimaanlage mit einem System zum automatischen Reinigen und Desinfizieren und entsprechendes Verfahren
Dispositif de climatisation avec assainissement d'air automatique et procédé correspondant

(30) Priority: 26.08.1999 IT MI991841
(43) Date of publication of application: 28.02.2001
(73) Proprietor: Beghelli S.p.A., 40050 Monteveglio, Bologna (IT)
(72) Inventor: Beghelli, Gian Pietro, 40050 Monteveglio (Bologna) (IT)
(74) Representative: De Gregori, Antonella

(56) References cited:
- DE-A- 19 603 623
- US-A- 3 824 770
- US-A- 5 286 447
- US-A- 5 447 693
- US-A- 5 788 569
- PATENT ABSTRACTS OF JAPAN vol. 1998, no. 14, 31 December 1998 (1998-12-31) -& JP 10 253096 A (KAWASAKI SETSUBI KOGYO KK), 25 September 1998 (1998-09-25)
- PATENT ABSTRACTS OF JAPAN vol. 015, no. 081 (M-1086), 25 February 1991 (1991-02-25) -& JP 02 302536 A (DAIKIN IND LTD), 14 December 1990 (1990-12-14)
- PATENT ABSTRACTS OF JAPAN vol. 018, no. 147 (M-1575), 11 March 1994 (1994-03-11) -& JP 05 322220 A (FUJITA CORP), 7 December 1993 (1993-12-07)

## Description

This invention refers to a self-sanitizing air-conditioning unit and to an automatic sanitizing process. Self-sanitizing air-conditioning units and processes are known from JP10253096 and from US5788569.

Air-conditioning units are known to be essentially composed of a pump which picks up a refrigerating fluid exiting from a condenser and conveys it to an evaporator held at a pressure which is higher than the condendenser pressure. The evaporator is in turn connected to the same condenser by a valve which may be of a different type but which in any case allows maintaining the pressure differential between the condenser and the evaporator.

In particular, the evaporator is constituted by a heat exchanger set into a duct through which the external air to be cooled is passed, before being conveyed into an environment whose conditions, in particular the temperature, are kept under control.

Such conventional air-conditioning units are notoriously subject to a contamination caused in their interior by bacterial or fungal growths as well as by the deposition of organic matter, dust or other impurities.

The contaminating phenomenon is particularly undesirable when it occurs inside the air duct, as in this case the contaminating materials are frequently entrained in the air crossing the duct whenever the unit is started up, and thus introduced into the air-conditioned environment. When this occurs, the contaminating materials may occasionally turn out to be harmful to the health of any persons dwelling in the air-conditioned premises, for instance by causing asthma or other unpleasant syndromes.

The purpose of this invention is therefore to eliminate the mentioned technical drawbacks, by creating a self-sanitizing air-conditioning unit capable of being sterilized in an essentially simple manner, so that the operation of the same fails to introduce some contaminating materials into the air-conditioned premises, which may turn out to be harmful or at any rate disagreeable to those dwelling inside.

Another purpose of the invention is to produce an air-conditioning unit capable of automatically sterilizing itself, without the need for an operator to initiate such an operation.

An additional purpose of the invention is to produce an air-conditioning unit incapable of introducing foreign harmful materials.

A further purpose of the invention is to produce a self-sanitizing air-conditioning unit of an essentially inexpensive, safe and reliable type.

Not the last purpose of the invention is to create a sanitizing process capable of being implemented by a self-sanitizing air-conditioning unit according to this invention.

These and other purposes according to this invention are achieved by producing a self-sanitizing air-conditioning unit according to claim 1.

Such an air-conditioning unit according to this invention implements a sanitizing process according to claim 4.

Other characteristics of this invention are further defined by the subsequent claims.

Further characteristics and advantages of the self-sanitizing air-conditioning unit and the relative sanitizing process according to this invention will become more clearly evident from the following description, offered for exemplifying an d non-limiting purposes, referred to the simplified attached drawings, in which:
Figure 1 offers a simplified view of an air-conditioning unit according to this invention, during a conditioning phase in which the conditioned air is introduced into an environment,
Figure 2 offers a simplified view of the same portion of the air-conditioning unit of Figure 1, during a sterilizing phase based on ozonizing its component parts, and in particular the evaporator,
Figure 3 shows the same portion of the air-conditioning unit of Figure 1, during a catalyzing phase preparing it for the subsequent sterilizing phase.

With reference to the mentioned figures, a portion of a self-sanitizing air-conditioning is shown in its overall form by the number 11.

The unit 11 comprises a boxy container 12, which houses a duct 14 through which the air to be treated is passed.

The duct 14 presents at one of its extremities an inlet through which the air to be treated, indicated by Fi, enters in its interior, a protective and encompassing grille 16.

A filter 18, a ventilator 20 and an evaporator 22 follow the grille inside the duct 14.

Behind the evaporator 22, the duct 14 is equipped with a second protective and encompassing grille 24 through which the treated air Fu exits.

Each of the two access openings to the duct 14 can also be shut off by a sliding diagram 26.

The duct 14 communicates with a sterilizing chamber 28 by two openings 30 placed at the opposite extremities of the duct 14. These openings 30 can be closed off by doors 32 hinged to the same container 12 and capable of being operated by stepping motors.

The inside of the chamber 28 houses an ozonizer 34, based on a known art, capable of producing and feeding ozone to the chamber 28 and a catalyzer 36, also of a known type, capable of eliminating the ozone which is harmful for human organisms.

The air-conditioning unit according to this invention also comprises an electronic control system 38 such as a PLC equipped with a timing device as a control element, which controls the opening and closing of the diaphragms 26 in addition to the electric motors for the doors 32, and also starts or interrupts the operation of the ozonizer 34 and the catalyzer 36.

While operating, the self-sanitizing air-conditioning unit according to this invention performs a sanitizing process essentially as follows.

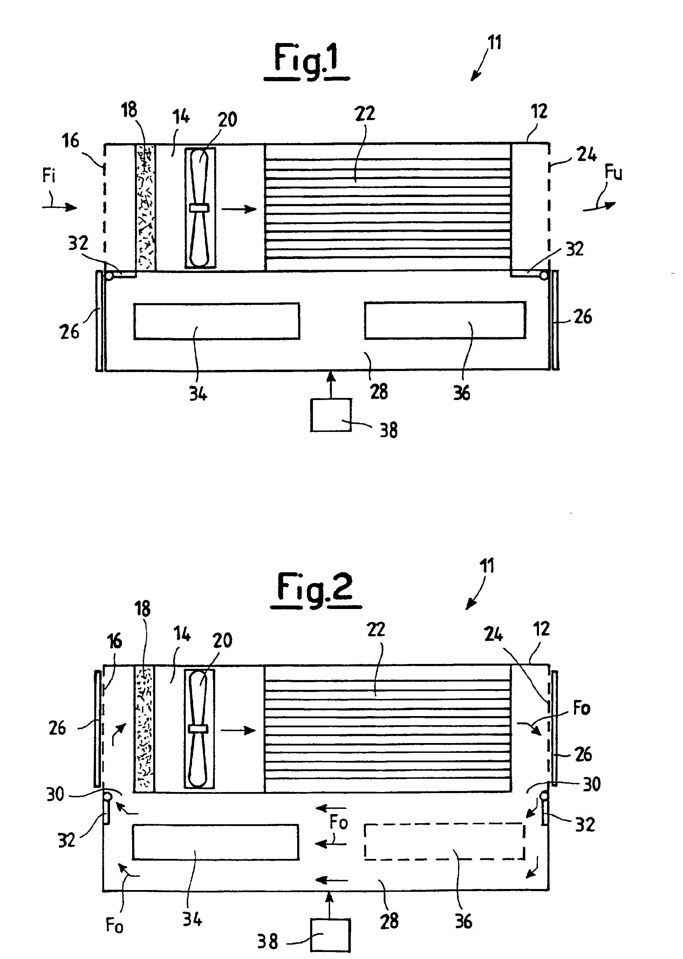

In a conditioning phase, as shown in Figure 1, both openings 30 are obscured by the doors 32, while both extremities of the duct 14 are open to allow the access of the air to be treated Fi and the exit of the treated air Fu from the duct.

In this configuration, the ventilator 20 draws air, which crosses the grille 16 and filter 18 and is then passed around the evaporator 22. The air is therefore cooled and conveyed to the environment to be conditioned, after passing the grille 24.

At pre-established intervals, the timer of the processor 38 automatically starts the sterilization of the unit 11.

When this happens, the processor 38 controls the opening of both of the openings 30, by rotating the doors 32, and the simultaneous closing of the extremities of the duct 14, by sliding the diaphragms 26.

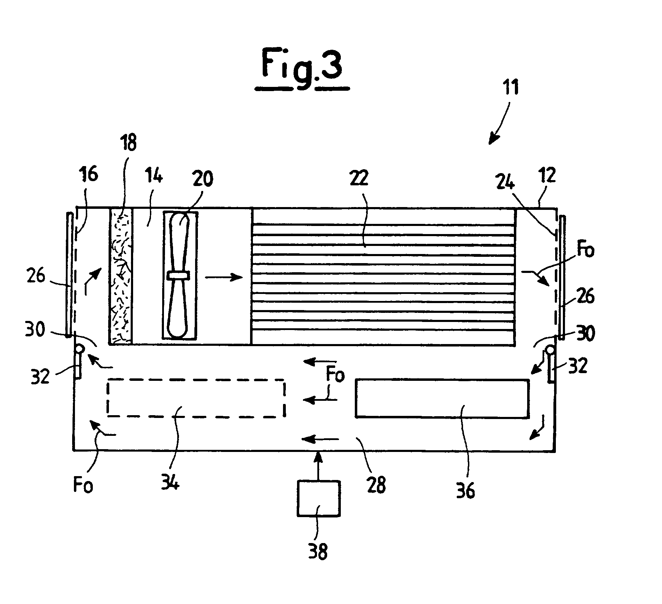

This starts the ozonizer 34 to produce ozone, which produces a mixture with air, indicated by Fo in Figures 2 and 3. The mixture Fo is then circulated through the unit 11, between the duct 14 and the chamber 28 of the ventilator 20. In this phase the fungi, bacterial floras, mildews and the like are attacked and destroyed by the ozone. The sterilizing phase has a duration which can be pre-set by acting on the electronic processor 38.

After the sterilizing phase, the processor 38 controls a catalyzing phase, which eliminates the ozone in the unit 11.

During the catalyzing phase shown in Figure 3, the openings 30 are still open and the mixture Fo contained in the unit 11 is still circulating between the duct 14 and the chamber 28. In this phase, however, the catalyzer 36 is actuated in place of the ozonizer 34, so as to totally eliminate the ozone after a certain operating time, and to return to clean air. This phase can also be pre-set by acting on the processor 38.

Finally, when the sterilizing and catalyzing phases are concluded, the processor 38 returns the unit to a conditioning mode by re-opening the extremities of the duct 14 and closing the openings 30 by using the doors 32.

It has in practice been shown that the self-sanitizing air conditioning unit and the relative sanitizing process according to this invention are particularly advantageous, as they allow eliminating the contaminating materials from the unit, without risking the immission into the conditioned environment of any materials differing from the contaminating ones, which may prove harmful to the organisms of the persons dwelling in the air-conditioned premises.

## Claims

1. A self-sanitizing air-conditioning unit (11) comprising at least one boxy container (12) crossed by the air to be treated, wherein a duct (14) is provided with a first opening and a second opening, at its respective first extremity and second extremity, through which the air to be treated enters (Fi) into the container (12) and exits (Fu) outside the container (12), said first and second openings being equipped with respective first and second protective and encompassing grilles (16, 24) and being closable by diaphragms (26), said duct (14) also containing at least one filter (18), positioned in series to said first grille (16), at least one ventilator (20) positioned in series to said filter (18) and at least one evaporator (22) positioned between said ventilator (20) and said second grille (24), **characterised in that** said boxy container (12) also includes a sterilizing chamber (28) containing at least one ozonizer (34) and a catalyzer (36), which are automatically operated by a control system (38), said sterilizing chamber (28) being directly in communication with said duct (14), by means of at least two passages (30), placed at the opposite extremities of said duct (14), when said ozonizer (34) or said catalyzer (36) is shut-in and said diaphragms (26) are actuated by said control system (38) to close said first and second openings of the duct (14), said at least two passages (30) being closed by respective doors (32), operated by said control system (38), when said ozonizer (34) and said catalyzer (36) are both shut-off and said diaphragms (26) are actuated by said control system (38) to open said first and second openings of the duct (14).

2. An air-conditioning unit (11) according to claim 1, **characterised in that** said doors (32) are hinged to said boxy container (12) and are operated by means of stepping motors.

3. An air-conditioning unit (11) according to claim 1, **characterised in that** said control system (38) is constituted by an electronic control device or PLC.

4. A sanitizing automatic process using the air-conditioning unit (11) according to claim 1, **characterised in that** said process comprises an ozonizing phase, during which the internal parts of said boxy container (12) are brought in contact with a mixture (Fo) containing at least air and ozone, and a catalyzing phase, during which said ozone is eliminated from said mixture (Fo).

## Patentansprüche

1. Selbsttätig reinigende und desinfizierende Klimaanlageneinheit (11) mit mindestens einem kastenartigen Behälter (12), der von der zu behandelnden Luft durchströmt wird, wobei ein Kanal (14) mit einer ersten Öffnung und einer zweiten Öffnung an seinem jeweiligen ersten Ende und zweiten Ende, durch die die zu behandelnde Luft in den Behälter (12) eintritt (Fi) und aus dem Behälter austritt (Fu), vorgesehen ist, wobei die erste und die zweite Öffnung mit jeweils einem ersten und einem zweiten Schutz- und Umgrenzungsgitter (16, 24) ausgestattet sind und durch Membranen (26) verschließbar sind, wobei der Kanal (14) auch mindestens einen Filter (18), der in Reihe mit dem ersten Gitter (16) angeordnet ist, mindestens einen Ventilator (20), der in Reihe mit dem Filter (18) angeordnet ist, und mindestens einen Verdampfer (22) enthält, der zwischen dem Ventilator (20) und dem zweiten Gitter (24) angeordnet ist, **dadurch gekennzeichnet, dass** der kastenartige Behälter (12) auch eine Sterilisierkammer (28) umfasst, die mindestens einen Ozonisator (34) und einen Katalysator (36) umfasst, die von einem Steuersystem (38) automatisch betrieben werden, wobei die Sterilisierkammer (28) mit dem Kanal (14) mittels mindestens zweier Durchgänge (30) in direkter Verbindung steht, die an den entgegengesetzten Enden des Kanals (14) angeordnet sind, wenn der Ozonisator (34) oder der Katalysator (36) eingeschaltet ist und die Membranen (26) von dem Steuersystem (38) betätigt sind, um die erste und die zweite Öffnung des Kanals (14) zu verschließen, wobei die mindestens zwei Durchgänge (30) durch jeweilige Türen (32) verschlossen sind, die von dem Steuersystem (38) betätigt werden, wenn der Ozonisator (34) und der Katalysator (36) beide ausgeschaltet sind und die Membranen (26) von dem Steuersystem (38) betätigt sind, um die erste und die zweite Öffnung des Kanals (14) zu öffnen.

2. Klimaanlageneinheit (11) nach Anspruch 1, **dadurch gekennzeichnet, dass** die Türen (32) an dem kastenartigen Behälter (12) angelenkt sind und mittels Schrittmotoren betätigt werden.

3. Klimaanlageneinheit (11) nach Anspruch 1, **dadurch gekennzeichnet, dass** das Steuersystem (38) durch eine elektronische Steuereinrichtung oder PLC gebildet sind.

4. Automatisches Reinigungs- und Desinfektionsverfahren, das die Klimaanlageneinheit (11) nach Anspruch 1 verwendet, **dadurch gekennzeichnet, dass** das Verfahren eine Ozonisierphase umfasst, während der die inneren Teile des kastenartigen Behälters (12) in Kontakt mit einem Gemisch (Fo) gebracht werden, das mindestens Luft und Ozon enthält, und eine Katalysierphase umfasst, während der das Ozon aus dem Gemisch (Fo) entfernt wird.

## Revendications

1. Unité de climatisation auto-assainissante (11), comprenant au moins un conteneur en forme de caisson (12) traversé par l'air à traiter, dans laquelle une conduite (14) est pourvue d'une première ouverture et d'une deuxième ouverture, respectivement au niveau de sa première extrémité et de sa deuxième extrémité, à travers lesquelles l'air à traiter entre (Fi) dans le conteneur et sort (Fu) du conteneur, lesdites première et deuxième ouvertures étant équipées respectivement de première et deuxième grilles de protection et de recouvrement (16, 24) et pouvant être fermées par des diaphragmes (26), ladite conduite (14) contenant également au moins un filtre (18) positionné en série par rapport à ladite première grille (16), au moins un ventilateur (20) positionné en série par rapport au dit filtre (18) et au moins un évaporateur (22) positionné entre ledit ventilateur (20) et ladite deuxième grille (24), **caractérisée en ce que** ledit conteneur en forme de caisson (12) comprend également une chambre de stérilisation (28) contenant au moins un ozoniseur (34) et un catalyseur (36), qui sont commandés de manière automatique par un système de commande (38), ladite chambre de stérilisation (28) étant directement en communication avec ladite conduite (14), au moyen d'au moins deux passages (30), situés aux extrémités opposées de ladite conduite (14), lorsque ledit ozoniseur (34) ou ledit catalyseur (36) est fermé et que lesdits diaphragmes (26) sont actionnés par ledit système de commande (38) pour fermer lesdites première et deuxième ouvertures de la conduite (14), lesdits au moins deux passages (30) étant fermés par des portes respectives (32), commandées par ledit système de commande (38), lorsque ledit ozoniseur (34) et ledit catalyseur (36) sont tous deux fermés et que lesdits diaphragmes (26) sont actionnés par ledit système de commande (38) pour ouvrir lesdites première et deuxième ouvertures de la conduite (14).

2. Unité de climatisation (11) selon la revendication 1, **caractérisée en ce que** lesdites portes (32) sont fixées par une charnière sur ledit conteneur en forme de caisson (12) et sont commandées au moyen de moteurs pas-à-pas.

3. Unité de climatisation (11) selon la revendication 1, **caractérisée en ce que** ledit système de commande (38) est constitué d'un dispositif de commande électronique ou d'un CPL.

4. Procédé d'assainissement automatique utilisant l'unité de climatisation (11) selon la revendication 1, **caractérisé en ce que** ledit procédé comprend une phase d'ozonisation, durant laquelle les éléments internes dudit conteneur en forme de caisson (12) sont mis en contact avec un mélange (Fo) contenant au moins de l'air et de l'ozone, et une étape de catalyse, durant laquelle ledit ozone est éliminé dudit mélange (Fo).
